# EUROPÄISCHE PATENTSCHRIFT

(11) **EP 2 319 552 B1**
(45) Veröffentlichungstag und Bekanntmachung des Hinweises auf die Patenterteilung: **08.01.2014**
(21) Anmeldenummer: 09075495.3
(22) Anmeldetag: 06.11.2009
(51) Int. Cl.: A61M 1/10, A61M 1/12

(54) **Blutpumpe**
Blood pump
Pompe à sang

(43) Veröffentlichungstag der Anmeldung: 11.05.2011
(73) Patentinhaber: Berlin Heart GmbH, 12247 Berlin (DE)
(72) Erfinder: Peters, Hans-Erhard, 10437 Berlin (DE); Müller, Jörg, 10439 Berlin (DE); Graichen, Kurt, 13189 Berlin (DE); Nüsser, Peter, 13347 Berlin (DE); Göllner, Manfred, 13086 Berlin (DE)
(74) Vertreter: Pfenning, Meinig & Partner GbR

(56) Entgegenhaltungen:
- WO-A1-00/64508
- WO-A1-02/066837
- WO-A1-2005/090791
- DE-A1- 19 625 300
- DE-A1-102006 036 948
- US-A1- 2007 100 196
- US-B1- 6 227 820
- US-B1- 6 368 083

## Beschreibung

Die Erfindung bezieht sich auf das Gebiet der Blutpumpen.

Unter einer Blutpumpe wird hier und im Folgenden eine Pumpe verstanden, die der Unterstützung oder Herstellung eines Blutstromes innerhalb eines menschlichen oder tierischen Körpers dient und sich zur Implantation im Brustraum eines Menschen oder Tieres außerhalb des Herzens eignet. Bei linksventrikularen Blutpumpen (left ventricular assist devices, LVAD) besteht eine Verbindung zwischen der linken Herzhälfte und einem Einlass der Blutpumpe sowie zwischen einem Auslass der Blutpumpe und der vom Herz abgehenden Aorta zur Unterstützung oder Herstellung des Blutkreislaufs durch den Körper (Körperkreislauf). Bei rechtsventrikularen Blutpumpen (RVAD) besteht eine Verbindung zwischen der rechten Herzhälfte und dem Lungenstamm, welcher zur linken und rechten Lungenarterie führt, (oder eine direkte Verbindung zwischen dem RVAD und der linken und/oder rechten Lungenarterie) zur Unterstützung oder Herstellung des Blutkreislaufs durch die Lunge (Lungenkreislauf). Innerhalb der Blutpumpe wird das Blut durch einen Hohlkörper geleitet, welcher Teil eines Pumpengehäuses ist oder in einem solchen Pumpengehäuse angeordnet ist. In dem Hohlkörper ist ein rotierendes Laufrad mit einer Beschaufelung zur Erzeugung eines Drucks und einer daraus resultierenden Strömung des Bluts vorgesehen. Sogenannte Totalherzpumpen (total artificial heart, TAH) beinhalten eine links- und eine rechtsventrikulare Blutpumpe zur Unterstützung oder Herstellung des gesamten Blutkreislaufs. Zur Herstellung der genannten Verbindungen zwischen der Blutpumpe und dem Herzen bzw. Blutgefäßen werden Verbindungsschläuche oder -rohre sowie ggf. Strömungskrümmer eingesetzt. Außerdem ist zur Energieversorgung und ggf. zur Steuerung der Blutpumpe wenigstens ein Kabelstrang erforderlich, der die Blutpumpe mit einem Energiespeicher und ggf. einer Steuerungseinheit verbindet.

Ein Hauptproblem bei der Implantation und der Verwendung solcher Blutpumpen, insbesondere von Totalherzpumpen, ist der Raumbedarf solcher Blutpumpen und der Verbindungsschläuche sowie des Kabelstrangs im Brustraum in der Nähe des Herzens.

Eine weitere Schwierigkeit liegt in der Gefahr der Zerstörung von Blutkörperchen (Hämolyse) durch die Blutpumpe, insbesondere an mechanischen Lagern des Laufrads, Engführungen oder abrupten Richtungsänderungen der Blutströmung durch die Blutpumpe sowie durch starke Druckgradienten innerhalb der Blutpumpe. Bei der Konstruktion von Blutpumpen werden zu diesem Zweck häufig mechanische Lager des Laufrads durch eine magnetische und/oder hydrodynamische Lagerungsvorrichtung ersetzt. Eine Radialpumpe mit teilweise aktiver magnetischer Lagerung ist in der DE 10 2006 036 948 gezeigt.

Ein zusätzliches Problem liegt darin, dass für den Lungenkreislauf ein wesentlich kleinerer Blutdruck hergestellt werden muss als für den Körperkreislauf, wobei aber durch beide Blutkreisläufe das gleiche Blutvolumen pro Zeit transportiert werden muss. Der von der Blutpumpe erzeugte Blutdruck hängt von der Drehzahl des Laufrads der Blutpumpe ab. Es stellt sich als schwierig heraus, eine Pumpe zu konstruieren, die sich zur Einstellung sehr unterschiedlicher Werte des Blutdrucks innerhalb eines Bereichs von etwa 5 mmHg bis etwa 150 mmHg bei einem stabilen konstanten, den physiologischen Bedingungen angepassten Volumenstrom zwischen 0 l/min bis 20 l/min eignet und sich auf diese Weise sowohl als RVAD als auch als LVAD einsetzen lässt bzw. sich zur Konstruktion einer Totalherzpumpe eignet.

Es ist somit Aufgabe der vorliegenden Erfindung, eine Blutpumpe sowie eine Totalherzpumpe vorzuschlagen, welche die oben genannten Probleme löst oder zumindest abmildert. Eine entsprechende Blutpumpe bzw. Totalherzpumpe soll also einen möglichst geringen Raumbedarf aufweisen und sich zu einer das Blut möglichst schonenden Unterstützung oder Herstellung eines Blutdrucks eignen. Außerdem soll sie sich zur Abdeckung eines breiten Bereichs des Blutdrucks bei einem möglichst konstanten Volumenfluss eignen.

Diese Aufgabe wird erfindungsgemäß gelöst durch Blutpumpen gemäß den unabhängigen Patentansprüchen.

Eine erfindungsgemäße Blutpumpe umfasst einen Hohlkörper, in dem ein Laufrad mit einer Beschaufelung vorgesehen ist zur Erzeugung eines axialen Vortriebs des Bluts entlang des Laufrads, sowie eine zumindest teilweise aktiv stabilisierte magnetische Lagerungsvorrichtung und eine hydrodynamische Lagerungsvorrichtung für das Laufrad, wobei das Laufrad mit einem außerhalb des Hohlkörpers befindlichen Motorstator in eine Rotation um eine Rotationsachse des Laufrads versetzbar ist und wobei der Hohlkörper einen Einlass zum Einströmen von Blut in den Hohlkörper in einer zur Rotationsachse im Wesentlichen parallelen Einströmrichtung und einen Auslass zum Ausströmen des Bluts aus dem Hohlkörper in einer Ausströmrichtung aufweist, wobei der Auslass gegen die Rotationsachse des Laufrads versetzt angeordnet ist zur Erzeugung eines von Null verschiedenen Ausströmwinkels zwischen der Einströmrichtung und der Ausströmrichtung.

Dabei wird unter dem Auslass eine Öffnung in einer Wandung des Hohlkörpers verstanden, wobei der Auslass in der Regel durch einen Anschlussstutzen nach außen weitergeführt wird.

Der Erfindung liegt der Gedanke zugrunde, durch den von Null verschiedenen Winkel zwischen der Einströmrichtung des Blutes und der Ausströmrichtung des Bluts einen möglichst geringen Raumbedarf der Blutpumpe einschließlich der notwendigen Verbindungsschläuche zu erzielen, wobei durch eine geeignete Wahl dieses Winkels der Auslass der implantierten Blutpumpe in Richtung des Blutgefäßes ausgerichtet werden kann, mit dem die Blutpumpe verbunden werden soll, also beispielsweise der Aorta, des Lungenstamms oder eines anderen Blutgefäßes. Auf diese Weise kann für diese Verbindung zwischen Blutpumpe und Blutgefäß eine besonders kurzer Verbindungsschlauch gewählt werden, da der Verbindungsschlauch möglichst geradlinig und auf direktem Wege zu dem Blutgefäß geführt werden kann und nicht entlang eines einen Umweg bildenden Bogens. Ferner wird der Einsatz von Strömungskrümmern zum Umlenken einer Strömungsrichtung des Blutes damit in der Regel überflüssig. Dieser Winkel liegt vorzugsweise in einem Bereich zwischen etwa 30° und etwa 150°, besonders bevorzugt in einem Bereich zwischen etwa 75° und etwa 105°, wobei bei einem Winkel von 90° das Blut die Blutpumpe in einem rechten Winkel zur Rotationsachse verlässt und bei einem Winkel von 0° die Ausströmung in axialer Richtung erfolgt.

Beim Einsatz von herkömmlichen axialen Blutpumpen sind in der Regel stark gekrümmte Verbindungsschläuche notwendig, da herkömmliche axiale Blutpumpen immer einen axialen Auslass aufweisen (d.h. der Winkel zwischen der Einströmrichtung und der Ausströmrichtung des Blutes beträgt etwa 0°).

Das Grundprinzip eines axialen Vortriebs des Bluts durch das Laufrad wird durch die erfindungsgemäße Blutpumpe beibehalten. Dies ist vorteilhaft, weil sich solche auch als Axialpumpen bezeichnete Blutpumpen, welche hauptsächlich eine axiale Krafteinwirkung auf das Blut ausüben und dieses somit hauptsächlich axial, also in Richtung der Rotationsachse entlang des Laufrads beschleunigen, das Blut besonders schonend fördern. Im Vergleich hierzu beschleunigen sogenannte Radialpumpen das Blut hauptsächlich radial zur Rotationsachse des Laufrads. Radialpumpen haben darüber hinaus meistens einen radialen Auslass, welcher häufig den Vorteil besonders kurzer Verbindungen zu Blutgefäßen mit sich bringt.

Die hier vorgeschlagene erfindungsgemäße Blutpumpe vereinigt in sich also sowohl den Vorteil einer schonenden Blutförderung einer Axialpumpe wie auch den Vorteil kurzer Verbindungsschläuche zwischen Blutpumpe und Blutgefäßen einer Radialpumpe und somit eines geringeren Platzbedarf sowie einer besseren Rotordynamik.

Die erfindungsgemäße Blutpumpe zeichnet sich außerdem durch den Wegfall eines dem Laufrad in Strömungsrichtung nachgelagerten Leitrads aus. Ein solches Nachleitrad dient in gewöhnlichen Axialpumpen mit axialem Auslass zur Umwandlung von Rotationsbewegungen des Blutes in einen zusätzlichen axialen Druckaufbau und somit einer Effizienzsteigerung der axialen Förderung des Blutes. Bei dem erfindungsgemäßen nicht-axialen Auslass trägt die Rotationsbewegung des Bluts zumindest teilweise auch zum durch die Blutpumpe erzeugten Blutdruck bei, welche durch das Weglassen des Nachleitrads vorteilhafterweise mitgenutzt werden kann. Außerdem wird durch das Weglassen des Nachleitrades die mechanische Belastung des Blutes durch eine Umlenkung des Bluts durch das Nachleitrad vermieden, wodurch die Gefahr der Blutschädigung weiter verringert wird.

Ein weiterer wesentlicher Vorteil, der durch das Einsparen des Nachleitrades erzielt wird, besteht in einer kleineren axialen Länge der Blutpumpe und somit einem reduzierten Raumbedarf der Blutpumpe.

Außerdem entfällt mit dem Nachleitrad das Problem von ungünstigen Anströmwinkeln des Nachleitrads bei bestimmten Arbeitspunkten der Blutpumpe. Wird das Nachleitrad nämlich unter einem ungünstigen Winkel angeströmt, können sogar durch das Nachleitrad Druckverluste entstehen. Zudem können sich lokale Druckschwankungen am Nachleitrad ausbilden, welche sich auch auf den Strömungsverlauf des Blutes am Laufrad ungünstig auswirken und eine stabile Lagerung des Laufrads insbesondere bei geringen Drehzahlen erschweren können.

Weiterhin ist vorgesehen, dass die Beschaufelung des Laufrads als Wendel ausgestaltet ist. Eine solche Wendel, welche ein- oder mehrgängig sein kann, ist vorzugsweise durchgängig und/oder verläuft über eine gesamte Länge des Laufrads und eignet sich auf diese Weise besonders gut zur Erzeugung eines schonenden, verwirbelungsarmen axialen Vortriebs des Blutes. Zudem lässt sich die Förderwirkung der Wendel über die Steigung der Wendel besonders leicht modifizieren. Selbstverständlich lassen sich aber auch andere Arten der Beschaufelung des Laufrads mit unterschiedlichen Steigungen realisieren. Vorzugsweise ist eine Außenkontur der Wendel zylinderförmig ausgestaltet.

Eine zumindest teilweise aktiv stabilisierte magnetische Lagerungsvorrichtung für das Laufrad, wie sie beispielsweise in WO 00/64030 beschrieben ist, eignet sich prinzipiell zur berührungsfreien Aufnahme radialer wie auch axialer Kräfte. Insbesondere hat sich eine aktive Stabilisierung der Axiallagerung (aktive Axialstabilisierung) als besonders vorteilhaft bei allen Drehzahlen herausgestellt. Zum Zweck einer aktiven Axialstabilisierung kann die magnetische Lagerungsvorrichtung in das Laufrad integrierte permanentmagnetische Elemente umfassen (das Laufrad enthält in der Regel außerdem auch permanentmagnetische Elemente für die Funktionalität als Motorrotor eines Elektromotors). Zusätzlich kann das magnetische Lagerungssystem für eine aktive Axialstabilisierung Ringspulen umfassen, welche mittels eines axialen Magnetflusses eine aktive Stabilisierung (Regelung) der axialen Lage des Laufrads ermöglichen. Diese Ringspulen sind unabhängig von einer Motorwicklung und dienen ausschließlich der aktiv stabilisierten axialen Lagerung des Laufrads. Die genannten Ringspulen können beispielsweise außerhalb des Hohlraums so angeordnet werden, dass sie diesen ringförmig umlaufen. Ferner kann die magnetische Lagerungsvorrichtung ein Sensorsystem zur Messung einer Position des Laufrads umfassen, insbesondere zur Feststellung einer Abweichung von einer axialen Sollposition, sowie eine mit dem Sensorsystem und den Ringmagneten verbundene Regeleinheit, welche den von den Ringmagneten erzeugten Magnetfluss gemäß der gemessenen axialen Position des Laufrads einstellt zum Korrigieren einer eventuellen Abweichung des Laufrads von der Sollposition. Weitere Details sind beispielsweise der oben genannten Druckschrift oder der Beschreibung spezieller Ausführungsformen der Erfindung weiter unten zu entnehmen.

In einer Weiterentwicklung umfasst die magnetische Lagerungsvorrichtung der Blutpumpe außerdem permanentmagnetische Lagerelemente für eine passive radiale Lagerung (passive Radialstabilisierung) des Laufrads. Diese permanentmagnetischen Lagerelemente können beispielsweise innerhalb des Hohlkörpers in unmittelbarer Nähe zu einer Zuströmseite und einer Abströmseite des Laufrads angeordnet sein, beispielsweise in einer Nabe des Laufrads, einem Leitrad oder einer Endschlussplatte des Hohlkörpers. Weitere Details sind wiederum der oben genannten Druckschrift oder der Beschreibung spezieller Ausführungsformen der Erfindung weiter unten zu entnehmen.

Die hydrodynamische Lagerung von Laufrädern in Blutpumpen ist prinzipiell bekannt. In einer Ausführungsform der Erfindung ist vorgesehen, die hydrodynamische Lagerungsvorrichtung des Laufrads wie in WO 02/66837 durch einen mit dem Laufrad verbundenen Stützring oder mehrere solcher Stützringe zu realisieren und auf diese Weise einen Ringspalt (oder mehrere Ringspalte) zwischen dem Stützring und einer Innenwandung des Hohlkörpers auszuformen für eine radiale Lagerung des Laufrads. Ein solcher Stützring, vorzugsweise als rotationssymmetrischer Hohlzylinder ausgeformt, lässt sich in unterschiedlichen Breiten ausführen und sich an einer beliebigen Stelle am Laufrad befestigen, um eine optimale Stabilisierung des Laufrads, insbesondere gegenüber einer axialen Verkippung des Laufrads, zu erzielen. Auf diese Weise können hydrodynamische und mechanische Unwuchten des Laufrads besonders wirkungsvoll ausgeglichen werden. Dies ist insbesondere dann vorteilhaft, wenn, wie weiter unten beschrieben wird, das Laufrad teilweise durch einen (spiralförmigen) Abflusskanal umlaufen wird. In diesem Fall kann ein entsprechender Stützring unmittelbar in Strömungsrichtung vor dem Abflusskanal zu einer Stabilisierung des Laufrads beitragen.

Eine Weiterentwicklung der Erfindung sieht vor, dass der Auslass des Hohlkörpers zwischen einer dem Einlass zugewandten Zuströmseite des Laufrads und einer dem Einlass abgewandten Abströmseite des Laufrads angeordnet ist. Auf diese Weise lassen sich besonders kompakte Ausführungsformen mit einer reduzierten Baulänge verwirklichen. Ferner stellt sich heraus, dass dies zu besonders guten Strömungseigenschaften der Blutpumpe und somit auch zu einer Stabilisierung des Laufrads beiträgt, wodurch sich der durch die Blutpumpe abdeckbare Druckbereich vergrößert. Vorzugsweise ist der Auslass in einer unmittelbaren Umgebung der Abströmseite des Laufrads angeordnet, um den axialen Vortrieb durch die Beschaufelung des Laufrads, welches vorzugsweise über eine Gesamtlänge seiner Mantelfläche hinweg beschaufelt ist, möglichst vollständig auszunutzen. Um dies zu steigern, erstreckt sich die Beschauflung des Laufrads vorzugsweise auch entlang des Auslasses, d.h. auf einer Höhe des Auslasses.

In einer Weiterentwicklung weist die Blutpumpe eine Rückschlussplatte auf, welche im Wesentlichen senkrecht zur Rotationsachse des Laufrads angeordnet ist zum Abschließen des Hohlkörpers. Im Fall, dass der Auslass, wie oben beschrieben, zwischen der Zuströmseite und der Abströmseite des Laufrads angeordnet ist, ist diese Abschlussklappe vorzugsweise in einer unmittelbaren Nähe zur Abströmseite des Laufrads angeordnet, um so strömungsfreie Toträume zwischen Laufrad und der Rückschlussplatte zu vermeiden. Solche Toträume bergen im Allgemeinen ein erhöhtes Risiko der Bildung von Blutgerinnseln und sind daher möglichst auszuschließen. In einer Weiterentwicklung ist die Rückschlussplatte als leicht zu öffnender Verschluss ausgestaltet zum einfachen Herstellen eines axialen Zugriffs in den Hohlkörper, etwa für einen leichteren Zusammenbau oder eine Justierung der Blutpumpe während des Montageprozesses.

In einer Ausführungsform ist vorgesehen, dass ein Innenradius des Hohlkörpers vergrößert ist zur Ausbildung eines das Laufrad tangential umlaufenden und in den Auslass mündenden Abflusskanals (Spiralgehäuse). Ein solcher Abflusskanal ermöglicht ein im Wesentlichen tangential zum Laufrad (genauer tangential zu einer Manteloberfläche des Laufrads) verlaufendes Abfließen des Bluts durch den Auslass aus dem Hohlkörper. Auf diese Weise können eine entsprechende tangentiale Strömungskomponente des Bluts und die dazugehörige kinetische Energie des Bluts beim Ausfließen aus dem Hohlkörper besonders gut und verlustarm erhalten bleiben und zur effizienten Erzeugung des Blutdrucks ausgenutzt werden. Eine tangential zur Manteloberfläche aber senkrecht zur Rotationsachse verlaufende Strömungskomponente des Bluts entsteht prinzipiell immer auch bei Axialpumpen, da der durch das Laufrad erzeugte Vortrieb neben der axialen Komponente auch eine Komponente senkrecht zur Rotationsachse aufweist. Insbesondere können durch einen solchen Abflusskanal Verwirbelungen mit entsprechenden Energieverlusten, wie sie bei einem einfachen radialen Auslass auftreten können, vermieden werden. Zudem verbessern sich auf diese Weise weiterhin die Strömungseigenschaften der Blutpumpe und somit auch die Stabilität des Laufrads, wodurch sich der durch die Blutpumpe abdeckbare Druckbereich weiter vergrößert. Außerdem kann gleichzeitig die mit den genannten Verwirbelungen einhergehende mechanische Belastung des Blutes weitestgehend vermieden werden.

Um eine möglichst hohe Effizienz der Blutförderung bei einer möglichst kompakten Bauform zu erzielen, erstreckt sich die Beschaufelung des Laufrads auch entlang des Abflusskanals, d.h. auf einer Höhe des Abflusskanals.

Eine Weiterentwicklung sieht vor, dass sich der Abflusskanal zum Auslass hin aufweitet und somit spiralförmig wie ein Spiralgehäuse ausgestaltet ist. Auf diese Weise wird eine kontinuierliche Reduktion der Strömungsgeschwindigkeit (bei gleich bleibendem Volumenstrom) zum Auslass hin und eine Reduktion von Wirbelbildungen erzielt und somit ein besonders schonendes Ausfließen des Blutes aus dem Hohlkörper gewährleistet. Durch die reduzierte Strömungsgeschwindigkeit des Blutes lässt sich zudem die durch die Blutpumpe erzeugte Druckerhöhung am Auslass im Übergang zur Auslasskanüle besonders wirkungsvoll übertragen. Typische Strömungsgeschwindigkeiten am Auslass der Blutpumpe liegen bei diesem Ausführungsbeispiel auf eine Größenskala unter 1 m/s.

In einer weiteren Ausführungsform ist vorgesehen, dass eine Mantelfläche des Laufrads, welche die Beschaufelung des Laufrads zwischen der Zu- und der Abströmseite trägt, im Wesentlichen zylinderförmig, kegelstumpfförmig oder kegelförmig ausgestaltet ist zur Erzeugung eines möglichst gleichmäßigen und wirbelfreien Vortriebs. Auf diese Weise lässt sich ein hauptsächlich axialer und somit für das Blut schonender Vortrieb gewährleisten. Es lässt sich aber auch durch einen zur Abströmseite des Laufrads hin zunehmenden Durchmesser des Laufrads eine radiale Beschleunigungskomponente erzielen.

Insbesondere in Kombination mit einer zylinderförmigen oder kegelstumpfförmigen Ausführung des Laufrads kann ein Vorleitrad vorgesehen sein, das auf der Zuströmseite des Laufrads angeordnet ist. Dieses Vorleitrad dient zum einen einer möglichst wirbelfreien und somit das Blut schonenden und von Energieverlusten möglichst freien Anströmung des Laufrads durch das Blut und kann außerdem eine stationäre Beschaufelung aufweisen, um eine Rotationsbewegung des Bluts um die Rotationsachse des Laufrads zu reduzieren und in einen axialen Vortrieb umzuwandeln zur weiteren Steigerung der Förderungsleistung der Blutpumpe. Vorzugsweise ist das Vorleitrad in einer unmittelbaren Nähe zur Zuströmseite des Laufrads angeordnet zur Vermeidung oder Reduzierung eines strömungsfreien Totraums zwischen dem Vorderleitrad und dem Laufrad. Ferner kann das Vorleitrad, wie oben bereits beschrieben, permanentmagnetische Lagerungselemente beinhalten, welche Bestandteile der magnetischen Lagerungsvorrichtung sind.

In einer Weiterentwicklung ist vorgesehen, dass die Blutpumpe eine Steuereinheit umfasst, die dazu eingerichtet ist, Drehzahlen des Laufrades einzustellen in einem Bereich zwischen 3000 U/min und 35000 U/min zur Erzeugung eines Blutdrucks am Auslass in einem Bereich zwischen 5 mmHg und 150 mmHg bei einem dem Bedarf angepassten Volumenstrom des Bluts. Auf diese Weise lässt sich je nach Strömungswiderstand des Lungen- bzw. Körperkreislauf ein Volumenstrom zwischen 0 I/min und 20 I/min einstellen.

Eine alternative Blutpumpe umfasst einen Hohlkörper, in dem ein Laufrad mit einer Beschaufelung vorgesehen ist zur Erzeugung eines axialen Vortriebs des Bluts entlang des Laufrads, wobei das Laufrad mit einem außerhalb des Hohlkörpers befindlichen Motorstator in eine Rotation um eine Rotationsachse des Laufrads versetzbar ist und wobei der Hohlkörper einen Einlass zum Einströmen von Blut in den Hohlkörper in einer zur Rotationsachse im Wesentlichen parallelen Einströmrichtung und einen Auslass zum Ausströmen des Bluts aus dem Hohlkörper in einer Ausströmrichtung aufweist, wobei der Auslass gegen die Rotationsachse des Laufrads versetzt angeordnet ist zur Erzeugung eines von Null verschiedenen Ausströmwinkels zwischen der Einströmrichtung und der Ausströmrichtung, wobei ein Innenradius des Hohlkörpers vergrößert ist zur Ausbildung eines das Laufrad tangential umlaufenden und in den Auslass mündenden, spiralförmigen Abflusskanals für ein im Wesentlichen tangential zum Laufrad verlaufendes Abfließen des Bluts aus dem Hohlkörper, wobei der Auslass des Hohlkörpers zwischen einer dem Einlass zugewandten Zuströmseite des Laufrads und einer dem Einlass abgewandten Abströmseite des Laufrads angeordnet ist.

Eine solche Blutpumpe kann zur Lagerung des Laufrads eine mechanische, hydrodynamische, eine magnetische, oder eine hybride Lagerungsvorrichtung enthalten. Ferner ist vorzugsweise eine die Beschaufelung tragende Mantelfläche des Laufrads im Wesentlichen zylinderförmig ausgestaltet für einen axialen Vortrieb des Blutes.

Zur Weiterentwicklung dieser alternativen Blutpumpe kommen alle oben aufgeführten und beschriebenen technischen Merkmale in Frage. Es ergeben sich die jeweils genannten Vorteile. Der Vollständigkeit halber seien die Merkmale hier noch einmal kurz aufgeführt. Für eine nähere Erläuterung sei auf die Ausführungen weiter oben verwiesen.

So kann die hydrodynamische Lagerungsvorrichtung des Laufrads als ein mit dem Laufrad verbundener Stützring ausgebildet sein zur Ausformung eines Ringspalts zwischen dem Stützring und einer Innenwandung des Hohlkörpers für eine radiale Lagerung des Laufrads. Es ist auch möglich, dass der Auslass des Hohlkörpers zwischen einer dem Einlass zugewandten Zuströmseite des Laufrads und einer dem Einlass abgewandten Abströmseite des Laufrads angeordnet ist. Es kann auch vorgesehen sein, dass ein Innenradius des Hohlkörpers vergrößert ist zur Ausbildung eines das Laufrad tangential umlaufenden und in den Auslass mündenden Abflusskanals für ein im Wesentlichen tangential zum Laufrad verlaufendes Abfließen des Bluts aus dem Hohlkörper. Ferner ist es möglich, dass sich dieser Abflusskanal zum Auslass hin aufweitet.

Ferner kann vorgesehen sein, dass die magnetische Lagerungsvorrichtung eine aktiv stabilisierte Axiallagerung umfasst.

Zudem ist es möglich, dass eine die Beschaufelung tragende Mantelfläche des Laufrads im Wesentlichen zylinderförmig, kegelförmig oder kegelstumpfförmig ausgestaltet ist. Die Beschaufelung des Laufrads ist als Wendel ausgestaltet.

Ferner kann ein Vorleitrad vorgesehen sein.

Schließlich kann die Blutpumpe eine Steuereinheit umfassen, die dazu eingerichtet ist, Drehzahlen des Laufrades einzustellen in einem Bereich zwischen 3000 U/min und 35000 U/min zur Erzeugung eines Blutdrucks an dem Auslass in einem Bereich zwischen 5 mmHg und 150 mmHg bei einem den physiologischen Bedingungen angepassten Volumenstrom.

In einer erfindungsgemäßen Totalherzpumpe ist vorgesehen, dass zwei Blutpumpen hier vorgeschlagener Art vorhanden sind, wobei eine erste Blutpumpe vorzugsweise als LVAD und eine zweite Blutpumpe als RVAD verwendet wird. Durch den Einsatz der hier beschriebenen Blutpumpen ist die Totalherzpumpe besonders raumsparend und lässt sich somit besonders leicht im Brustraum am Herz anordnen.

In einer Ausführungsform ist vorgesehen, dass die Laufräder der beiden Blutpumpen der Totalherzpumpe auf einer gemeinsamen Rotationsachse angeordnet sind, wodurch eine besonders einfache Konstruktion und Montage ermöglicht wird. Ferner erlaubt dies eine vorteilhaft schlanke Form der Totalherzpumpe, wodurch eine Implantation in den Brustraum erleichtert wird.

In einem Ausführungsbeispiel sind die Laufräder der beiden Blutpumpen zu einem einzigen, gemeinsamen Laufrad fest miteinander verbunden, wobei die Hohlräume der beiden Blutpumpen zu einem gemeinsamen Hohlkörper (Gehäuse) zusammengefasst sind. Dies erlaubt einen axial besonders kurzen Aufbau der Totalherzpumpe. Außerdem ist auf diese Weise ein einfachere Lagerung möglich, da das gemeinsame Laufrad weniger Freiheitsgrade aufweist als zwei einzelne Laufräder.

In einem weiteren Ausführungsbeispiel ist vorgesehen, dass zwischen dem Laufrad der ersten Blutpumpe und dem Laufrad der zweiten Blutpumpe ein Lagerbock vorhanden ist, in dem zumindest Teile der Lagervorrichtung der ersten und/oder der zweiten Blutpumpe integriert sind.

Im Folgenden werden spezielle Ausführungsformen der Erfindung anhand von Figuren 1 bis 4 näher beschrieben. Gleiche Bezugszeichen bezeichnen gleiche gegenständliche Merkmale. Es zeigt:
- Figur 1: eine schematische Darstellung eines Längs- schnitts durch eine Blutpumpe hier vorge- schlagener Art,
- Figur 2: eine schematische Darstellung eines Längs- schnitts durch eine Blutpumpe hier vorge- schlagener Art,
- Figur 3: eine schematische Darstellung eines Quer- schnitts durch einen Hohlkörper einer Blut- pumpe hier vorgeschlagener Art,
- Figur 4: eine schematische Darstellung eines teil- weise aufgeschnittenen Hohlkörpers einer Blutpumpe hier vorgeschlagener Art,
- Figur 5: eine schematische Darstellung einer Total- herzpumpe hier vorgeschlagener Art mit ei- nem einzigen Laufrad und
- Figur 6: eine schematische Darstellung einer Total- herzpumpe hier vorgeschlagener Art mit zwei einzelnen Laufrädern.

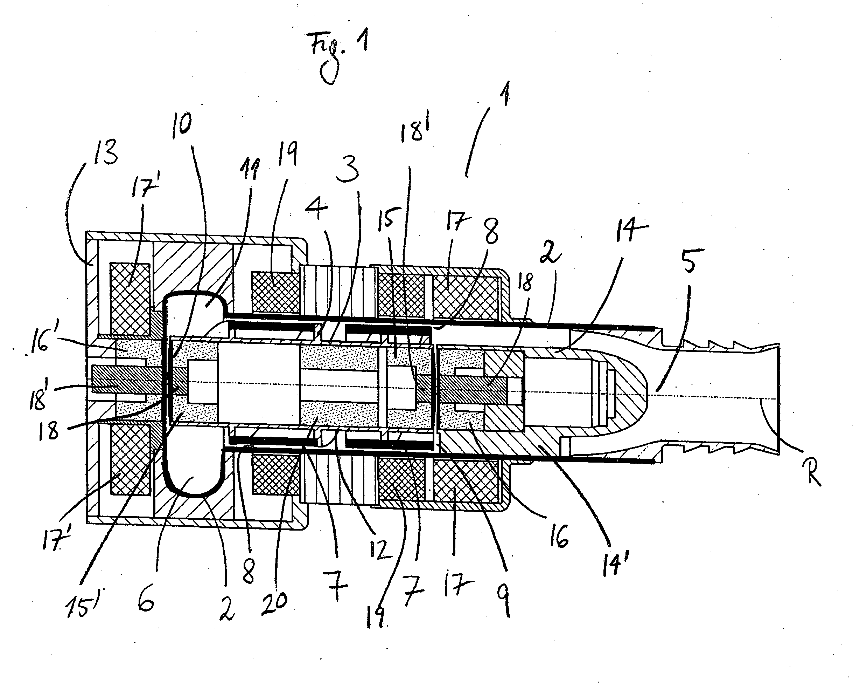

In Figur 1 ist eine schematische Darstellung eines Längsschnitts durch eine Blutpumpe 1 hier vorgeschlagener Art schematisch dargestellt. Die Blutpumpe 1 umfasst einen Hohlkörper 2 (als durchgezogene dicke Linie dargestellt), in dem ein Laufrad 3 mit einer Beschaufelung 4 vorgesehen ist. Ferner weist der Hohlkörper 2 einen Einlass 5 zum Einströmen von Blut in einer zu einer Rotationsachse R (gestrichelt dargestellt) parallelen Einströmrichtung und einen Auslass 6 zum Ausströmen des Bluts in einer Ausströmrichtung, welche senkrecht zur Schnittebene verläuft. Demnach ist in diesem Ausführungsbeispiel der Auslass relativ zur Rotationsachse R in einem rechten Winkel versetzt angeordnet zur Erzeugung eines von Null verschiedenen Ausströmwinkels α von α = 90° zwischen der Einströmrichtung und der Ausströmrichtung.

Der Auslass 6 des Hohlkörpers 2 ist zwischen einer dem Einlass zugewandten Zuströmseite 9 des Laufrads 3 und einer dem Einlass abgewandten Abströmseite 10 des Laufrads 3 angeordnet. Ein Innenradius des Hohlkörpers 2 dient zur Ausbildung eines das Laufrad 3 tangential umlaufenden und in den Auslass 6 mündenden Abflusskanals 11 für ein im Wesentlichen tangential zum Laufrad 3 verlaufendes Abfließen des Bluts aus dem Hohlkörper 2.

Ferner ist eine hydrodynamische Lagerungsvorrichtung vorgesehen, welche als zwei mit dem Laufrad 3 verbundene Stützringe 7 ausgestaltet ist, zur Ausformung von zwei Ringspalten 8 zwischen den Stützringen 7 und einer Innenwandung des Hohlkörpers 2 für eine radiale Lagerung des Laufrads 3.

Eine die Beschaufelung 4 tragende Mantelfläche 12 des Laufrads 3 ist zylinderförmig ausgeformt, könnte aber genauso gut kegelstumpfförmig oder kegelförmig ausgestaltet sein. Eine axiale Abmessung (Länge) L des Laufrads ist größer gewählt als ein Durchmesser D des Laufrads auf der Abströmseite des Laufrads. Die Beschaufelung des Laufrads ist durch eine Steigung gekennzeichnet, die zum Auslass 6 hin zunimmt. Auf diese Weise wird ein für das Blut besonders schonender axialer Vortrieb bis zum Abflusskanal 11 hin ermöglicht. Die Beschaufelung 4 erstreckt sich axial ganz (in anderen Ausführungsformen teilweise) in den Abflusskanal 11 und den Ausfluss 6.

In unmittelbarer Nähe zur Zuströmseite 9 des Laufrads 3 ist ein Vorleitrad 14 vorgesehen, welches mit einer Beschaufelung 14' ausgestattet ist.

Die Blutpumpe umfasst ferner eine teilweise aktiv stabilisierte Lagerungsvorrichtung, welche eine aktiv stabilisierte magnetischen Axiallagerung sowie eine passive magnetische Radiallagerung beinhaltet. Die magnetische Lagerungsvorrichtung umfasst zunächst zwei in dem Laufrad an der Zuströmseite und an der Abströmseite angeordnete Permanentmagnete 15, 15'. Ferner dienen zwei weitere, zu diesen entgegengesetzt (anziehend) gepolte permanente Lagermagnete 16, 16' der Ausbildung des passiven magnetischen Radiallagers, welches dafür sorgt, dass das Laufrad 3 in einer radialen Sollposition zwischen dem Vorleitrad 14 und der Rückschlussplatte 13 gehalten wird. Ferner sind für die aktiv stabilisierte magnetische Axiallagerung zwei Ringspulen 17, 17' außerhalb des Hohlkörper 2 vor und hinter dem Laufrad 3 so angeordnet, dass sie den Hohlkörper 2 ringförmig umlaufen zur Erzeugung eines axialen Magnetflusses. Ferner umfasst die magnetische Lagerungsvorrichtung ein Sensorsystem, welches in dem Vorleitrad 14 und/oder der Rückschlussplatte 13 sowie in dem Laufrad 3 integrierte Abstandssensoren 18, 18' umfasst zur Messung von Spaltbreiten zwischen dem Laufrad 3 und dem Vorleitrad 14 bzw. der Rückschlussplatte 13, sowie eine mit den Abstandssensoren 18, 18' und den Ringmagneten verbundene Regeleinheit (hier nicht dargestellt), welche den von den Ringmagneten erzeugten Magnetfluss gemäß der gemessenen axialen Position des Laufrads einstellt zum Korrigieren einer eventueller Abweichung des Laufrads von einer axialen Sollposition.

Schließlich sind eine den Hohlkörper umlaufende Motorwicklung 19 und ein im Laufrad integrierter Motormagnet 20 vorgesehen, welcher alternierend radial magnetisiert ist, zum Antreiben des Laufrads 3.

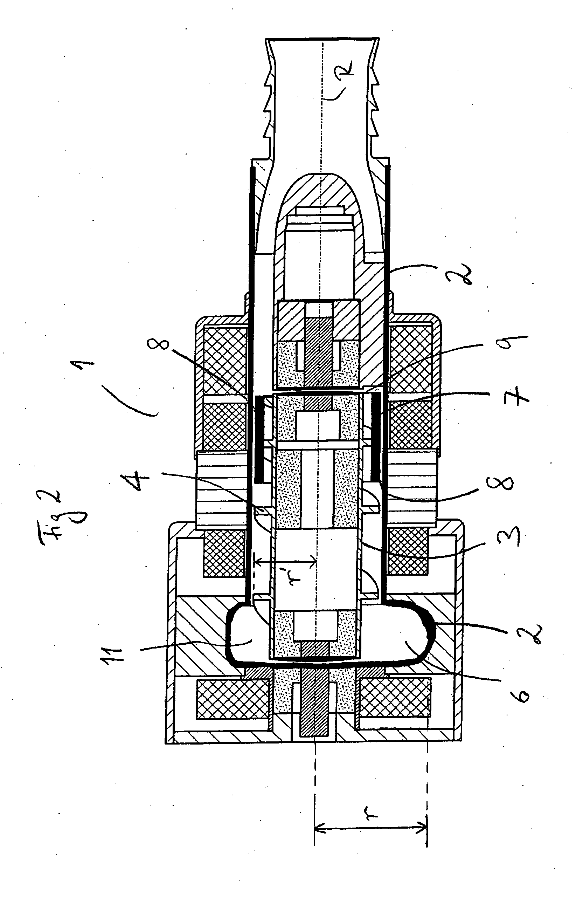

In Figur 2 ist eine schematische Darstellung eines Längsschnitts durch eine Blutpumpe 1 hier vorgeschlagener Art gezeigt, welche sich von der anhand Figur 1 beschriebenen Blutpumpe nur durch eine veränderte hydrodynamische Lagerungsvorrichtung unterscheidet. In dem in Figur 2 gezeigten Beispiel ist diese als ein einzelner mit dem Laufrad 3 verbundener Stützring 7 ausgestaltet zur Ausformung eines einzelnen Ringspaltes 8 zwischen dem Stützring 7 und einer Innenwandung des Hohlkörpers 2 für eine radiale Lagerung des Laufrads 3.

Außerdem ist ein Radius r des Hohlkörpers 2 auf einer Höhe des Abflusskanals 11 dargestellt, wobei sich dieser Radius zum Auslass hin vergrößert zur Ausformung eines spiralförmigen Abflusskanals 11, welcher sich zum Auslass hin erweitert. Ein Radius der Laufradbeschaufelung ist als r' bezeichnet. Es gilt r' < r.

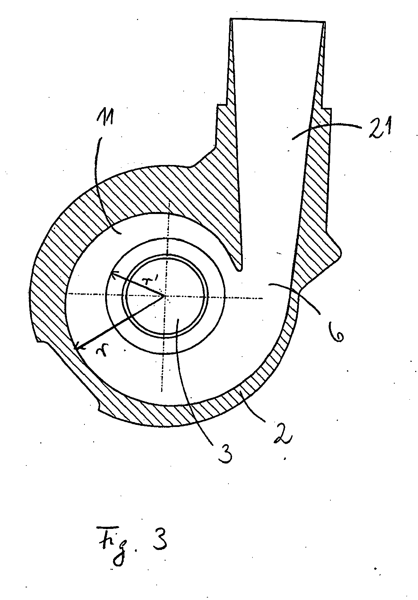

Figur 3 zeigt eine schematische Darstellung eines Querschnitts durch einen Hohlkörper 2 einer Blutpumpe 1 gemäß Figur 1 oder Figur 2. Der Querschnitt verläuft senkrecht zur Rotationsachse R durch den Abflusskanal 11 des Hohlkörpers 2 der Blutpumpe 1. Der Hohlkörper 2 weist einen Radius r auf, der im Vergleich zu einem Radius r' des Hohlkörpers auf einer Höhe der Zuströmseite 3 des Laufrads 3 vergrößert ist, zur Ausbildung des Abflusskanals 11. Der Abflusskanal 11 weitet sich in seinem Verlauf zum Abfluss 6 hin spiralförmig auf und bildet auf diese Weise ein Spiralgehäuse aus. Der Abfluss 6 ist in einen Anschlussstutzen 21 nach außen fortgesetzt, der sich nach außen weiter aufweitet zur Verkleinerung der Strömungsgeschwindigkeit des Bluts.

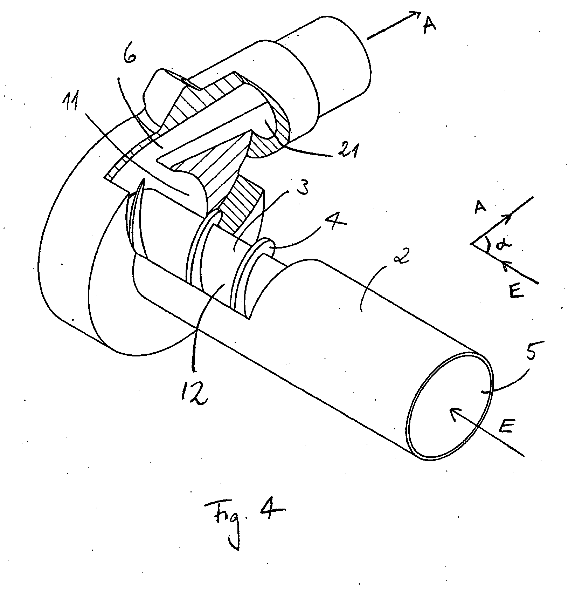

Figur 4 zeigt eine schematische Darstellung eines teilweise aufgeschnittenen Hohlkörpers 2 einer Blutpumpe 1 gemäß Figur 3. Zu erkennen ist wiederum der Hohlkörper 2 mit dem Einlass 5 zum Einströmen von Blut in einer Einströmrichtung, die durch den mit E bezeichneten Pfeil gekennzeichnet ist, einem zu einem Auslassstutzen 21 verlängerten tangentialen Auslass 6 zum Ausströmen des Bluts in einer Ausströmrichtung, die durch den mit A bezeichneten Pfeil gekennzeichnet ist und die in einem rechten Winkel zur Einströmrichtung E verläuft.

In dem Hohlkörper ist das zylinderförmige axiale Laufrad 3 angeordnet, wobei Figur zusätzlich beispielhaft die Überdeckung eines Teils des Laufrads durch den spiralförmigen Auslasskanal zeigt. Der spiralförmige Auslasskanal 11 verläuft tangential zum Laufrad 3, mündet in dem Auslass 6 und bildet auf diese Weise eine Spiralkammer (Spiralgehäuse) aus.

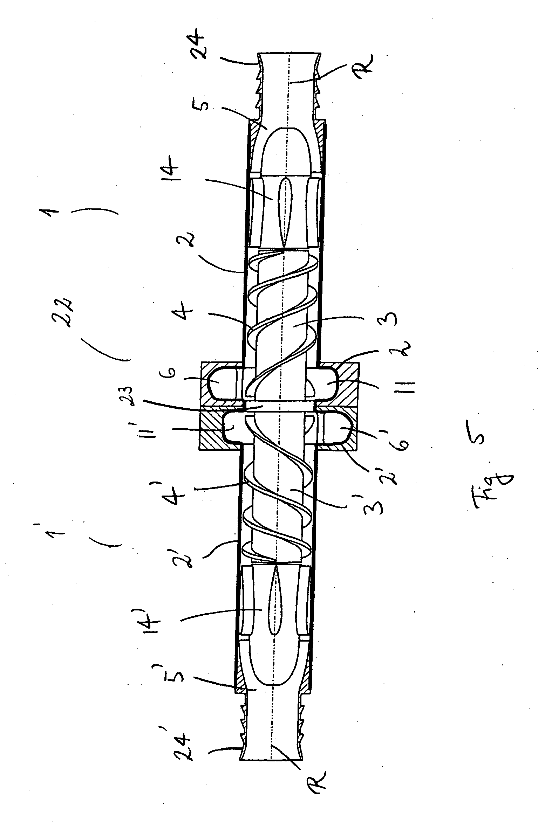

In Figur 5 ist eine Ausführungsform einer Totalherzpumpe 22 hier vorgeschlagener Art schematisch dargestellt. Sie umfasst zwei Blutpumpen 1, 1' hier vorgeschlagener Art, deren Hohlkörper 2, 2' axial zu einem gemeinsamen Hohlkörper verbunden sind. Dieser weist an seinen beiden Enden zwei Einlässe 5, 5' auf zum Einströmen von Blut aus dem Lungenkreislauf bzw. dem Körperkreislauf, so dass die rechte Blutpumpe 1 als RVAD und die linke Blutpumpe 1' LVAD vorgesehen ist. Die beiden Laufräder 3, 3' der beiden Blutpumpen 1, 1' sind axial zu einem gemeinsamen Laufrad fest miteinander verbunden. Das Blut ist durch eine entsprechende Ausgestaltung der Beschaufelung 4, 4' des gemeinsame Laufrads 3, 3' zu einer Mitte des gemeinsamen Hohlkörpers 2, 2' hin axial antreibbar, an welchem zwei spiralförmige Auslasskanäle 11, 11' (Spiralkammern) ausgeformt sind, welche jeweils in einen Auslass 6, 6' münden zum tangentialen (rechtwinkligen) Ausströmen des Bluts aus dem gemeinsamen Hohlkörper 2, 2'.

Die Beschaufelung 4, 4' des Laufrads ist zur Erzeugung zweier unterschiedlicher Werte des Blutdrucks an den beiden Auslässen 6, 6' ausgestaltet. Zu diesem Zweck ist die Steigung der wendelförmigen Beschaufelung entsprechend angepasst.

Zwischen den beiden Auslasskanälen 11, 11' besteht ein Verbindungsspalt 23 zwischen dem gemeinsamen Hohlkörper 2, 2' und dem gemeinsamen Laufrad 3, 3'. Der Verbindungsspalt 23 kann möglichst eng ausgestaltet werden, um eine Leckströmung des Blutes zwischen den Hohlkörpern 3, 3' der ersten und der zweiten Blutpumpe 1, 1' möglichst zu reduzieren.

Ferner weist die Totalherzpumpe 22 an den beiden Einlässen jeweils eine Olive 24, 24' (Verbindungsstück) auf zum Anschließen eines Verbindungsschlauches.

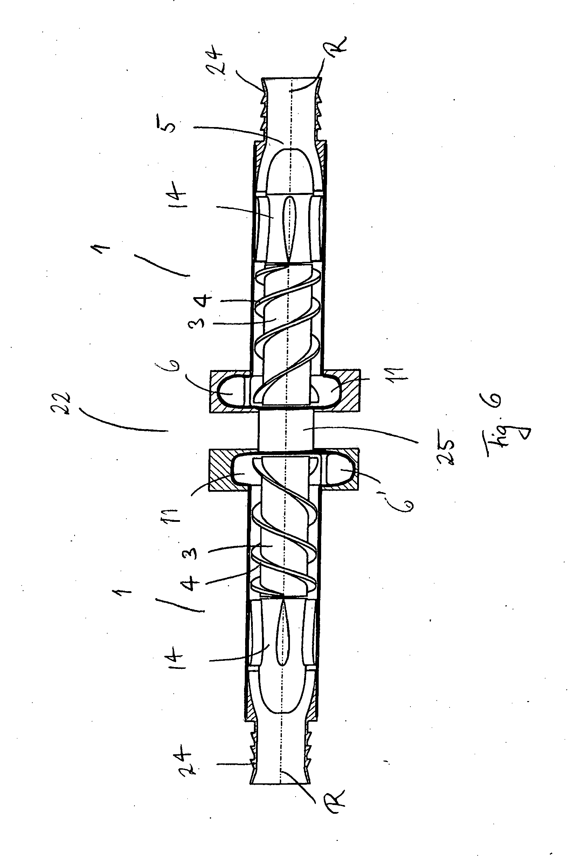

In Figur 6 ist eine Ausführungsform einer Totalherzpumpe 22 hier vorgeschlagener Art schematisch dargestellt. Sie umfasst zwei Blutpumpen 1, 1' hier vorgeschlagener Art, deren Hohlkörper 2, 2' axial zueinander ausgerichtet und über einen Lagerbock 25 fest miteinander verbunden sind. Der Lagerbock beinhaltet Teile der Lagerungsvorrichtungen (z.B. permanentmagnetische Lagermagnete zur Axiallagerung) der beiden Blutpumpen 1, 1' zu Lagerung der beiden Laufräder 3, 3'. Diese sind mechanisch nicht miteinander verbunden und somit unabhängig voneinander um die gemeinsame Rotationsachse R drehbar. Die beiden Einlässe 5, 5' sind zum Einströmen von Blut aus dem Lungenkreislauf bzw. dem Körperkreislauf vorgesehen, so dass wie im vorherigen Ausführungsbeispiel die rechte Blutpumpe 1 als RVAD und die linke Blutpumpe 1' LVAD bestimmt ist. Das Blut ist durch eine entsprechende Wahl der Drehzahl und/oder durch eine unterschiedliche Ausgestaltung der Beschaufelung 4, 4' der beiden Laufräder 3, 3' in Richtung des Lagerbocks 25 axial antreibbar. Ferner sind jeweils an den Abströmseiten 10, 10' der beiden Laufräder 3, 3' spiralförmige Auslasskanäle 11, 11' (Spiralkammern) vorgesehen, welche jeweils in einen Auslass 6, 6' münden zum tangentialen (rechtwinkligen) Ausströmen des Bluts aus den Hohlkörpern 2, 2'.

## Patentansprüche

1. Blutpumpe (1), umfassend einen Hohlkörper (2), in dem ein Laufrad (3) mit einer Beschaufelung (4) vorgesehen ist zur Erzeugung eines axialen Vortriebs des Bluts entlang des Laufrads (3), sowie eine zumindest teilweise aktiv stabilisierte magnetische Lagerungsvorrichtung (15, 15', 16, 16', 17, 17', 18, 18') und eine hydrodynamische Lagerungsvorrichtung (7) für das Laufrad, wobei das Laufrad mit einem außerhalb des Hohlkörpers befindlichen Motorstator (19) in eine Rotation um eine Rotationsachse (R) des Laufrads (3) versetzbar ist und wobei der Hohlkörper (2) einen Einlass (5) zum Einströmen von Blut in den Hohlkörper (2) in einer zur Rotationsachse (R) im Wesentlichen parallelen Einströmrichtung (E) und einen Auslass (6) zum Ausströmen des Bluts aus dem Hohlkörper (2) in einer Ausströmrichtung (A) aufweist, wobei der Auslass (6) gegen die Rotationsachse (R) des Laufrads (3) versetzt angeordnet ist zur Erzeugung eines von Null verschiedenen Ausströmwinkels (α) zwischen der Einströmrichtung (E) und der Ausströmrichtung (A), **dadurch gekennzeichnet, dass** die Beschaufelung (4) des Laufrads (3) als Wendel ausgestaltet ist.

2. Blutpumpe (1) aus Anspruch 1, **dadurch gekennzeichnet, dass** die hydrodynamische Lagerungsvorrichtung (7) des Laufrads als ein mit dem Laufrad verbundener Stützring ausgebildet ist zur Ausformung eines Ringspalts (8) zwischen dem Stützring (7) und einer Innenwandung des Hohlkörpers für eine radiale Lagerung des Laufrads (3).

3. Blutpumpe (1) nach einem der Ansprüche 1 oder 2, **dadurch gekennzeichnet, dass** der Auslass (6) des Hohlkörpers (2) zwischen einer dem Einlass (5) zugewandten Zuströmseite (9) des Laufrads (3) und einer dem Einlass (5) abgewandten Abströmseite (10) des Laufrads (3) angeordnet ist.

4. Blutpumpe (1) nach einem der vorangehenden Ansprüche, **dadurch gekennzeichnet, dass** ein Innenradius (r) des Hohlkörpers (2) vergrößert ist zur Ausbildung eines das Laufrad tangential umlaufenden und in den Auslass (6) mündenden Abflusskanals (11) für ein im Wesentlichen tangential zum Laufrad verlaufendes Abfließen des Bluts aus dem Hohlkörper (2).

5. Blutpumpe (1) nach dem vorangehenden Anspruch, **dadurch gekennzeichnet, dass** sich der Abflusskanal (11) zum Auslass (6) hin aufweitet.

6. Blutpumpe nach einem der vorangehenden Ansprüche 4 oder 5, **dadurch gekennzeichnet, dass** sich die Beschaufelung (4) des Laufrads (3) auch entlang des Abflusskanals (11) erstreckt.

7. Blutpumpe (1) nach einem der vorangehenden Ansprüche, **dadurch gekennzeichnet, dass** eine die Beschaufelung tragende Mantelfläche (12) des Laufrads im Wesentlichen zylinderförmig, kegelförmig oder kegelstumpfförmig ausgestaltet ist.

8. Blutpumpe (1) nach einem der vorangehenden Ansprüche, **dadurch gekennzeichnet, dass** sich die Beschaufelung (4) des Laufrads (3) über eine Gesamtlänge des Laufrads (3) hinweg erstreckt.

9. Blutpumpe (1) nach einem der vorangehenden Ansprüche, **dadurch gekennzeichnet, dass** die magnetische Lagerungsvorrichtung eine aktiv stabilisierte Axiallagerung (15, 15', 17, 17', 18, 18') umfasst.

10. Blutpumpe (1) nach Anspruch 9, **dadurch gekennzeichnet, dass** die Axiallagerung (15, 15', 17, 17', 18, 18') Ringspulen umfasst, welche den Hohlkörper (2) ringförmig umlaufen und von einer Motorwicklung der Blutpumpe (1) unabhängig sind, zur Erzeugung eines axialen Magnetflusses für die aktiv stabilisierte Axiallagerung des Laufrads (3).

11. Blutpumpe (1) nach einem der vorangehenden Ansprüche, **dadurch gekennzeichnet, dass** ein Vorleitrad (14) vorgesehen ist.

12. Totalherzpumpe (22), umfassend zwei Blutpumpen (1) nach mindestens einem der vorangehenden Ansprüche.

13. Totalherzpumpe (22) aus Anspruch 12, **dadurch gekennzeichnet, dass** die Laufräder (3) der beiden Blutpumpen (1) auf einer gemeinsame Rotationsachse (R) angeordnet sind.

14. Totalherzpumpe (22) nach einem der Ansprüche 12 oder 13, **dadurch gekennzeichnet, dass** die Laufräder (3, 3') der beiden Blutpumpen (1, 1') fest miteinander verbunden sind zur Ausbildung eines gemeinsamen Laufrads, wobei die Hohlkörper (2, 2') der beiden Blutpumpen (1, 1') zu einem gemeinsamen Hohlkörper zusammengefasst sind.

## Claims

1. Blood pump (1), comprising a hollow body (2), in which an impeller (3) with a blading (4) is provided for producing an axial propulsion of the blood along the impeller (3), as well as an at least partly actively stabilised magnetic bearing device (15, 15', 16, 16', 17, 17', 18, 18') and a hydrodynamic bearing device (7) for the impeller, wherein the impeller may be set into a rotation about a rotation axis (R) of the impeller (3) with a motor stator (19) located outside the hollow body, and wherein the hollow body (2) comprises an inlet (5) for the flow of blood into the hollow body (2) in an inflow direction (E) which is essentially parallel to the rotation axis (R), and an outlet (6) for the outflow of the blood out of the hollow body (2) in an outflow direction (A), wherein the outlet (6) is arranged offset to the rotation axis (R) of the impeller (3) for producing an outflow angle (α) between the inflow direction (E) and the outflow direction (A), which is different to zero,
**characterised in that**
the blading (4) of the impeller (3) is designed as a spiral.

2. Blood pump (1) of claim 1, **characterised in that** the hydrodynamic bearing device (7) of the impeller is designed as a support ring which is connected to the impeller, for forming an annular gap (8) between the support ring (7) and an inner wall of the hollow body for a radial bearing of the impeller (3).

3. Blood pump (1) according to one of claims 1 or 2, **characterised in that** the outlet (6) of the hollow body (2) is arranged between an upstream-side (9) of the impeller (3), said upstream-side facing the inlet (5), and a downstream-side (10) of the impeller (3), said downstream-side being away from the inlet (5).

4. Blood pump (1) according to one of the preceding claims, **characterised in that** an inner radius (r) of the hollow body (2) is enlarged for forming a discharge channel (11) which runs tangentially around the impeller and runs out into the outlet (6), for a flowing-away of the blood out of the hollow body (2), running essentially tangentially to the impeller.

5. Blood pump (1) according to the preceding claim, **characterised in that** the discharge channel (11) widens towards the outlet (6).

6. Blood pump according to one of the preceding claims 4 or 5, **characterised in that** the blading (4) of the impeller (3) also extends along the discharge channel (11).

7. Blood pump (1) according to one of the preceding claims, **characterised in that** a peripheral surface (12) of the impeller, said peripheral surface carring the blading, is designed in an cylinder-shaped manner, cone-shaped manner or truncated-cone-shaped manner.

8. Blood pump (1) according to one of the preceding claims, **characterised in that** the blading (4) of the impeller (3) extends along the entire length of the impeller (3).

9. Blood pump (1) according to one of the preceding claims, **characterised in that** the magnetic bearing device comprises an actively stabilised axial bearing (15, 15', 17, 17', 18, 18').

10. Blood pump (1) according to claim 9, **characterised in that** the axial bearing (15, 15', 17, 17', 18, 18') comprises toroidal coils which run annularly around the hollow body (2) and are independent from a motor winding of the blood pump (1), for producing an axial magnetic flux for the actively stabilised axial bearing of the impeller (3).

11. Blood pump (1) according to one of the preceding claims, **characterised in that** an inlet guide vane (14) is provided.

12. Total artificial heart (22) comprising two blood pumps (1) according to at least one of the preceding claims.

13. Total artificial heart (22) from claim 12, **characterised in that** the impellers (3) of the two blood pumps (1) are arranged on a common rotational axis (R).

14. Total artificial heart (22) according to one of claims 12 or 13, **characterised in that** the impellers (3, 3') of the two blood pumps (1, 1') are firmly connected with each other for forming a common impeller, wherein the hollow bodies (2, 2') of the two blood pumps (1, 1') are combined to form a common hollow body.

## Revendications

1. Pompe sanguine (1) comprenant un corps creux (2) dans lequel est prévue une roue (3) avec un aubage (4), pour produire un entraînement axial du sang le long de la roue (3), ainsi qu'un dispositif de support magnétique (15, 15', 16, 16', 17, 17', 18, 18') au moins partiellement stabilisé activement et un dispositif de support hydrodynamique (7) pour la roue, dans laquelle la roue peut être mise en rotation autour d'un axe de rotation (R) de la roue (3), à l'aide d'un stator de moteur (19) situé à l'extérieur du corps creux, et dans laquelle le corps creux (2) comporte une admission (5) pour l'entrée du sang dans le corps creux (2), dans une direction d'admission (E) essentiellement parallèlement à l'axe de rotation (R), ainsi qu'une évacuation (6) pour la sortie du sang hors du corps creux (2), dans une direction d'évacuation (A), dans laquelle l'évacuation (6) est agencée de façon décalée par rapport à l'axe de rotation (R) de la roue (3), pour produire un angle d'évacuation (α) différent de zéro entre la direction d'admission (E) et la direction d'évacuation (A),
**caractérisée en ce que**
l'aubage (4) de la roue est conçu en spirale.

2. Pompe sanguine (1) selon la revendication 1, **caractérisée en ce que** le dispositif de support hydrodynamique (7) de la roue est conçu comme un anneau de support relié à la roue, pour la formation d'une fente annulaire (8) entre l'anneau de support (7) et une paroi intérieure du corps creux, pour un support radial de la roue (3).

3. Pompe sanguine (1) selon la revendication 1 ou 2, **caractérisée en ce que** l'évacuation (6) du corps creux (2) est agencée entre un côté arrivée (9) de la roue (3) tourné vers l'admission (5) et un côté sortie (10) de la roue (3) détourné de l'admission (5).

4. Pompe sanguine (1) selon l'une des revendications précédentes, **caractérisée en ce qu'**un rayon intérieur (r) du corps creux (2) est agrandi, pour la formation d'un canal d'évacuation (11) entourant tangentiellement la roue et débouchant dans l'évacuation (6), pour un écoulement du sang hors du corps creux (2) de façon essentiellement tangentielle à la roue.

5. Pompe sanguine (1) selon la revendication précédente, **caractérisée en ce que** le canal d'évacuation (11) s'élargit vers l'évacuation (6).

6. Pompe sanguine selon l'une des revendications précédentes 4 ou 5, **caractérisée en ce que** l'aubage (4) de la roue (3) s'étend également le long du canal d'évacuation (11).

7. Pompe sanguine (1) selon l'une des revendications précédentes, **caractérisée en ce qu'**une surface d'enveloppe (12) de la roue portant l'aubage est conçue essentiellement cylindrique, conique ou en forme de cône tronqué.

8. Pompe sanguine (1) selon l'une des revendications précédentes, **caractérisée en ce que** l'aubage (4) de la roue (3) s'étend sur une longueur totale de la roue (3).

9. Pompe sanguine (1) selon l'une des revendications précédentes, **caractérisée en ce que** le dispositif de support magnétique comprend un palier axial stabilisé (15, 15', 17, 17', 18, 18').

10. Pompe sanguine (1) selon la revendication 9, **caractérisée en ce que** le palier axial (15, 15', 17, 17', 18, 18') comprend des bobines toroïdales entourant le corps creux (2) de façon annulaire et étant indépendantes d'un enroulement de moteur de la pompe sanguine (1), pour produire un flux magnétique axial pour le palier axial stabilisé activement de la roue (3).

11. Pompe sanguine (1) selon l'une des revendications précédentes, **caractérisée en ce qu'**il est prévu une roue de pré-regulation (14).

12. Pompe cardiaque totale (22), comprenant deux pompes sanguines (1) selon l'une au moins des revendications précédentes.

13. Pompe cardiaque totale (22) selon la revendication 12, **caractérisée en ce que** les roues (3) des deux pompes sanguines (1) sont agencées sur un même axe de rotation (R).

14. Pompe cardiaque totale (22) selon l'une des revendications 12 ou 13, **caractérisée en ce que** les roues (3, 3') des deux pompes sanguines (1, 1') sont reliées fixement l'une à l'autre pour former une roue commune, dans laquelle les corps creux (2, 2') des deux pompes sanguines (1, 1') sont réunis en un corps creux commun.
